# EUROPEAN PATENT SPECIFICATION

(11) **EP 4 578 391 B1**
(45) Date of publication and mention of the grant of the patent: **02.09.2026**
(21) Application number: 23307440.0
(22) Date of filing: 30.12.2023
(51) Int. Cl.: A61B 5/361, A61B 5/00, G16H 50/20

(54) **COMPUTER DEVICE FOR REAL-TIME ANALYSIS OF ELECTROGRAMS**
RECHNERVORRICHTUNG ZUR ECHTZEITANALYSE VON ELEKTROGRAMMEN
DISPOSITIF INFORMATIQUE POUR L'ANALYSE EN TEMPS RÉEL D'ÉLECTROGRAMMES

(43) Date of publication of application: 02.07.2025
(73) Proprietor: Substrate HD, 13006 Marseille (FR)
(72) Inventor: DEMARCY, Thomas, 13006 Marseille (FR); SILVA, Santiago, 13006 Marseille (FR)
(74) Representative: Plasseraud IP

(56) References cited:
- FR-A1- 3 119 537
- SUNDARARAJAN PRATHIC ET AL: "Automatic Diagnosis of Cardiac Disease from Twelve-Lead and Reduced-Lead ECGs Using Multilabel Classification", 2021 COMPUTING IN CARDIOLOGY (CINC), CREATIVE COMMONS, vol. 48, 13 September 2021 (2021-09-13), pages 1 - 4, XP033999351, DOI: 10.23919/CINC53138.2021.9662899
- RUIZ ALEJANDRO PASOS ET AL: "The great multivariate time series classification bake off: a review and experimental evaluation of recent algorithmic advances", JOURNAL OF DATA MINING AND KNOWLEDGE DISCOVERY, vol. 35, no. 2, 1 March 2021 (2021-03-01) - 1 March 2021 (2021-03-01), pages 401 - 449, XP037376162, ISSN: 1384-5810, DOI: 10.1007/S10618-020-00727-3

## Description

The invention concerns a computer device for real-time analysis of electrograms.

### Technical Field

The field of atrial fibrillation has been rapidly developing in the past 5 years.

### Background

The detection of cardiac areas that promote atrial fibrillation includes both off-line computing devices using software such as Topera and CardioInsight, and real-time computing devices using software such as CARTO.

The Topera software aims to reconstruct the electrical activation of the atria of the heart. Signal acquisition is carried out using a "basket catheter" (a catheter that is deployed over the entire atrium). The analysis is performed in delayed time, more than 2 minutes after the start of the acquisition. This solution is inconvenient because this type of catheter is difficult to insert and because the contact of the electrodes is not guaranteed. The analysis duration is very long, and the reconstruction does not allow high-definition maps to be obtained, and even fails in the case of complex electrical activation (which represents 70% of atrial fibrillation cases) due to an overly simplistic reconstruction.

The CardioInsight software aims to reconstruct the electrical activity of the heart using a multi-electrode electrocardiogram measurement vest on the patient's skin. The patient wears the vest before the operation, the data is analyzed and accessible during the operation. The analysis is done in delayed time (more than 15 minutes) after the beginning of the acquisition. This solution has the disadvantage of a very long computation time, which imposes a deferred time making it difficult to set up (the patient is required to come a few days ahead and wear the vest so that the data can be extracted before the operation), and a very high cost. In addition, the reconstruction does not allow high-definition maps because the measurements are set up too far apart, and fails in the case of complex electrical activation because of an overly simplistic reconstruction.

The CARTO software published by Biosense & Webster allows the implementation of algorithms to detect cardiac arrhythmias.

The CFAE (Complex Fractionated Atrial Electrograms) algorithm calculates the number of deflection points (variation of the sign of the derivative) in the signals and produces color maps in real-time. The practitioner then interprets these complex maps to determine places of interest. This algorithm is not very specific and relatively simple.

The Ensite software published by Abbott Laboratories allows the implementation of algorithms to detect cardiac arrhythmias.

The Ripple algorithm (Ripple Mapping) is a module of the CARTO software that allows to reproduce the electrical activity of the atria after a first catheter passage. Both the amplitude and the propagation of electrical waves are made visible through this module. This results in maps that are extremely complex for the practitioner to understand. While this may work in simple cases, the analysis fails in the case of complex electrical activation because the maps are too difficult to interpret.

The Applicant has long used a solution which differs from all existing solutions in that it relies on the determination of spatiotemporal dispersion in electrograms. This measurement was discovered and discussed in the article by Seitz et al. "AF Ablation Guided by Spatiotemporal Electrogram Dispersion Without Pulmonary Vein Isolation: A Wholly Patient-Tailored Approach", J Am Coll Cardiol. 2017 Jan, 69 (3) 303-321, https://doi.org/10.1016/j.jacc.2016.10.065 and was the object of a patent family based on French patent application FR1463232.

The Applicant followed this effort with an improved system and filed a patent family based on French patent application FR1852850 which disclosed using two interlinked methods in order to perform real-time detection of cardiac areas that promote atrial fibrillation. Embodiments of this application use a classifier arranged to apply two classification models to electrogram data. One model is faster to apply and less precise than the other, such that it is used to alert the practitioner of the potential interest of a cardiac region.

### Summary

The further works of the Applicant led to filing a patent family based on patent application FR2101234 which discloses a device for determining the presence of spatiotemporal dispersion in electrograms by combining a gradient boosted tree and a convolutional neural network.

In all of the Applicant's inventions, the spatiotemporal dispersion is obtained by comparing the signals of pairs of electrodes. However, this method is rigid as its training is complicated when introducing new types of catheter which have a differing number of electrodes, or when the spatial distribution of the electrodes themselves is not sufficiently similar between different types of catheters.

The invention aims at improving the situation.

According to a first aspect, a computer device for analysis of electrograms is provided which comprises a data storage arranged to receive electrogram signals each originating from one of a plurality of electrodes of a catheter and catheter electrode distance data, a random convolutional kernel-based feature extractor arranged to receive electrogram signals associated with the electrodes of a catheter for a given timecode and to return an electrode feature vector for each electrogram signal, an input generator arranged to receive the electrogram feature vectors output by said random convolutional kernel-based feature extractor and catheter electrode distance data relating to the catheter from which these electrogram signals originate, to associate each electrode with at least the three closest electrodes of the catheter based on said catheter electrode distance data, and to return an input vector in which the electrode feature vectors of each electrode is grouped with the electrode feature vectors of its associated electrodes and the corresponding catheter electrode distance data, and a gradient-boosted based machine learning module trained on data comprising sets of input vectors labelled with a value indicating for each electrode if its electrogram signal exhibits spatiotemporal dispersion. The computer device is arranged to receive electrogram signals associated and electrode distance date associated with a timecode, to feed them to said input generator, to use the resulting input vectors as input for said gradient-boosted based machine learning module, and to return a dispersion value for each electrode.

This device is advantageous because it allows a spatiotemporal measurement to be made which better takes into account the 3D repartition of the electrodes and to better associates each electrode to its neighbors in order to run the gradient boosted tree. Furthermore, the use of a random convolutional kernel-based feature extractor has proven both more efficient than conventional measurements and more adaptable to fit all types of catheters. Finally, this also makes the device more open to evolution as the inputs are catheter agnostic.

In various embodiments, the computer device may present one or more of the following features:
- the computer device operates in real-time on electrogram signals and the electrode distance data acquired in real-time,
- the input generator is arranged to associate each electrode with at least the four closest electrodes of the catheter based on said catheter electrode distance data,
- the gradient boosted machine learning module is an XGB, and
- the device is further arranged to determine a color associated to the spatiotemporal dispersion values for each electrogram signal, the computer device further comprising a display arranged to output, for each electrode, the color associated to the spatiotemporal dispersion value determined for the corresponding electrogram signal.

According to a second aspect, a method for determining spatiotemporal dispersion in electrograms is provided. This computer implemented method for analyzing electrogram signals and corresponding electrode distance data comprises the following operations:
a) Applying a random convolutional kernel-based feature extractor to said electrogram signals to return an electrode feature vector for each input electrogram signal,
b) For each electrode feature vector of operation a), based on the electrode distance data, associating electrode feature vectors of the at least three electrodes closest to the electrode from which is derived said each electrode feature vector of operation a), along with the corresponding electrode distance data,
c) For each input vector, applying a gradient boosted machine learning module trained on data comprising sets of input vectors labelled with a value indicating for each electrode if its electrogram signal exhibits spatiotemporal dispersion, and returning the corresponding values.

In various embodiments, this method may present one or more of the following features:
- the method is performed in real-time on electrogram signals and the electrode distance data acquired in real-time,
- operation b) is arranged to associate each electrode with at least the four closest electrodes of the catheter based on said catheter electrode distance data,
- the gradient boosted machine learning module is an XGB, and
- the method further comprises d) determining a color associated to the spatiotemporal dispersion values for each electrogram signal, and outputting, for each electrode, the color associated to the spatiotemporal dispersion value determined for the corresponding electrogram signal.

According to a third aspect, a computer program is provided. The computer program includes instructions for adapting an apparatus to perform the method of any one of the embodiments of the second aspect.

According to a fourth aspect, a carrier containing a computer program that includes instructions for adapting an apparatus to perform the method of any one of the embodiments of the second aspect is provided. In some embodiments, the carrier is one of an electronic signal, optical signal, radio signal, or computer readable storage medium.

According to a fifth aspect, an apparatus is provided. The apparatus includes processing circuitry and a memory containing instructions executable by the processing circuitry that causes the apparatus to perform the method of any one of the embodiments of the second aspect.

Other features and advantages of the invention will readily appear in the following description of the drawings, which show exemplary embodiments of the invention and on which:
- Figure 1 shows a general diagram of a computer device according to an embodiment,
- Figure 2 shows a generic view of a computer program used in the embodiment of Figure 1,
- Figure 3 shows a visual return on a display based on spatiotemporal dispersion values, and
- Figure 4 shows a flow chart illustrating a process according to an embodiment.

The drawings and the following description are comprised for the most part of positive and well-defined features. As a result, they are not only useful in understanding the invention, but they can also be used to contribute to its definition, should the need arise.

Persistent atrial fibrillation remains a therapeutic challenge. While an increasingly larger number of patients is eligible for catheter ablation, the optimal ablation strategy for persistent atrial fibrillation remains elusive. STAR-AF2 (Verma A, Jiang C-y, Betts TR et al. "Approaches to catheter ablation for persistent atrial fibrillation". New England Journal of Medicine 2015; 372:1812-1822) concluded that performing additional ablation lesions beyond pulmonary vein isolation (PVI) does not lead to improved long-term outcomes.

By contrast, some observational studies (see Jadidi AS, Lehrmann H, Keyl C et al. "Ablation of persistent atrial fibrillation targeting low-voltage areas with selective activation characteristics", Circulation: Arrhythmia and Electrophysiology 2016;9:e002962 and Seitz J, Bars C, Théodore G et al. "AF ablation guided by spatiotemporal electrogram dispersion without pulmonary vein isolation: a wholly patient-tailored approach", Journal of the American College of Cardiology 2017;69:303-321) have suggested that non-PVI lesions targeting regions harboring electrogram abnormalities are beneficial to patients with persistent atrial fibrillation.

In particular, the latter study showed the results of which supported the application of radio-frequency energy in atrial regions exhibiting the dispersion of multipolar electrograms. Other studies (Narayan SM, Krummen DE, Shivkumar K, Clopton P, Rappel W-J, Miller JM. "Treatment of atrial fibrillation by the ablation of localized sources: CONFIRM (Conventional Ablation for Atrial Fibrillation With or Without Focal Impulse and Rotor Modulation) trial", Journal of the American College of Cardiology 2012;60:628-636) instead performed advanced signal analysis and provided a mechanistic-based display to guide operators towards atrial fibrillation drivers.

Regardless of the electrogram abnormalities or analyses, there are large differences in experience and stage in the learning curve between centers attempting to implement similar electrogram-based mapping approaches.

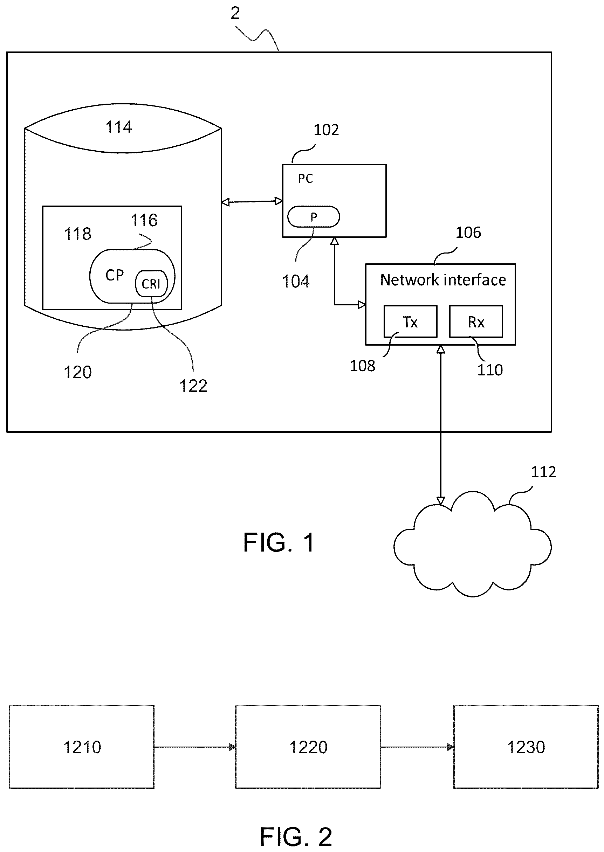

Figure 1 shows a general diagram on an embodiment of the computer device according to the invention.

The computer device shown on Figure 1 as a block diagram of an apparatus 2 (*e.g.,* a network node, connected device, and the like), according to an embodiment. As shown in Figure 1 the apparatus may comprise: processing circuitry (PC) 102, which may include one or more processors (P) 104; a network interface 106 comprising a transmitter (Tx) 108 and a receiver (Rx) 110 for enabling the apparatus to transmit data to and receive data from other computing devices connected to a network 112 (e.g., an Internet Protocol (IP) network) to which network interface 106 is connected; and a local storage unit (a.k.a., "data storage system") 114, which may include one or more non-volatile storage devices and/or one or more volatile storage devices.

In embodiments where PC 102 includes a programmable processor, a computer program product (CPP) 116 may be provided. CPP 116 includes a computer readable medium (CRM) 118 storing a computer program (CP) 120 comprising computer readable instructions (CRI) 122. CRM 118 may be a non-transitory computer readable medium, such as, magnetic media (e.g., a hard disk), optical media, memory devices (e.g., random access memory, flash memory), and the like.

Processors 104 include any means known for performing automated calculus, such as CPUs, GPUs, CPUs and/or GPUs grids, remote calculus grids, specifically configured FPGAs, specifically configured ASICs, specialized chips such as SOCs or NOCs, AI specialized chips, etc.

In an embodiment, data storage 114 stores electrogram signals (for example after they have been digitalized and split at regular time intervals, such that all electrogram signals have the same length, e.g., 3 seconds) along with catheter electrode distance data. The electrogram signals may be based on real-time acquisition of electrograms by a catheter during a procedure, or they may be based on differed-time. The electrogram signals are the result of the sensing by the electrodes. The same applies to the catheter electrode distance data. The catheter electrode distance data may also be fixed, *e.g.* be based on theoretical or manufacturer provided distance data between the electrodes of a given catheter model. As will appear below, the electrogram signals are transformed into electrode feature vectors. The electrode feature vectors are also stored in data storage 114. The database may further contain a training set of electrode feature vectors, corresponding catheter electrode distance data, each labelled with a value indicating whether they exhibit spatiotemporal dispersion, *i.e.,* whether they are associated with atrial fibrillation or not. In the example described herein, the Applicant has established a database of almost 500000 such elements which form its training dataset (almost 21h30m of signals). Electrogram signals may also be post-treated in order to be added to the training dataset for retraining or continuous training of the gradient boosted tree. The results return by apparatus 2 may be used by the practitioner to mark (or otherwise have the associated position returned in an automatic manner) the corresponding locations on a patient's heart, which leads to a map of a heart with zones to be treated in view of atrial fibrillation represented as dots. In some embodiments, this map can be generated automatically based on the apparatus 2 outputs.

In the example described herein, the data storage 114 may be realized in any way suitable, that is by means of a hard disk drive, a solid-state drive, a flash memory, a memory embedded in a processor, a distant storage accessible in the cloud, etc. Data storage 114 may also store any transitory data which may be generated in the course of executing the invention, as well as maps resulting from the operation of the apparatus 2, possibly combined with practitioner made annotations.

Figure 2 shows a exemplary schematic of computer program 120.

The computer program (CP) 120 comprises three main modules which work hand-in-hand:
- A random convolutional kernel-based feature extractor 1210,
- An input generator 1220, and
- A gradient-boosted based machine learning module 1230.

In the example described herein, the random convolutional kernel-based feature extractor 1210 is based on the framework Rocket described in the article by Dempster et al. "ROCKET: exceptionally fast and accurate time series classification using random convolutional kernels", Data Min Knowl Disc 34, 1454-1495 (2020), https://doi.org/10.1007/s10618-020-00701-z.

ROCKET is a machine learning algorithm for feature extraction. It generates random convolutional kernels (random length, weights, dilation, and padding) and random thresholds (called bias) which can transform multivariate time series into high-dimensional feature vectors. It can capture complex patterns and relationships.

The Applicant has also found that a multivariate alternative of ROCKET, named MiniROCKET can be even more efficient, as the multivariate random feature generation can capture relationships between specific tracks, and allows for better real-time performance. MiniROCKET is described in the article by Dempster et al. "MINIROCKET: A Very Fast (Almost) Deterministic Transform for Time Series Classification", KDD '21: Proceedings of the 27th ACM SIGKDD Conference on Knowledge Discovery & Data MiningAugust 2021, Pages 248-257, https://doi.org/10.1145/3447548.3467231.

The random convolutional kernel-based feature extractor 1210 receives the electrogram signals associated with a chosen timecode in the data storage 114, *i.e.* the electrogram signals which have been obtained at the same time from the electrodes of a given catheter, and it returns, for each electrogram track (or electrogram signal), an electrode feature vector.

In the example described herein, the input generator 1220 merely acts as a wrapper for the gradient-boosted based machine learning module 1230. In other words, the input generator 1220 uses the data processed by the random convolutional kernel-based feature extractor 1210 and the electrode distance data, and it packages into an input vector format which has been used for the training of the gradient-boosted based machine learning module 1230. In the example described herein, for a given electrode for which a spatiotemporal dispersion value is sought, the input vector comprises the following data:
- The electrode feature vector of the given electrode.
- The electrode feature vectors of the electrodes which are closest to the given electrode, based on the electrode distance data associated with the corresponding electrogram signals. The Applicants' research has shown that the best results are obtained with 4 neighbor electrodes or more, and that the best tradeoff for real-time performance is obtained for exactly 4 neighbor electrodes. However, the results have also shown that 3 neighbor electrodes can be sufficient. The advantage of choosing the closest electrodes based on electrode distance data is that it allows to use the electrodes which are actually the closest to the given electrode. Also, if there is a problem with one of the electrode tracks, it remains possible to have a working input vector, as opposed to the past systems of the Applicant in which the electrode pairings were monolithic and far-field effects are observed when inter-electrode distance is too large.
- The electrode distance data.

In some embodiments, the input generator 1220 could be considered as a mere input mode of the gradient-boosted based machine learning module 1230. The gradient-boosted based machine learning module 1230 may be any boosted tree implementation, such as XGB (see the article by Chen et al. "XGBoost: A Scalable Tree Boosting System", In Proceedings of the 22nd ACM SIGKDD International Conference on Knowledge Discovery and Data Mining (KDD '16), Association for Computing Machinery, New York, NY, USA, 785-794. https://doi.org/10.1145/2939672.2939785), or LightGBM (see the article by Ke et al. "LightGBM: A highly efficient gradient boosting decision tree", Advances in Neural Information Processing Systems, 30, 3146-3154).

The output of the gradient-boosted based machine learning module 1230 is, for each input vector, a value reflecting the spatiotemporal dispersion of the corresponding electrogram signal. As mentioned above, the gradient-boosted based machine learning module 1230 in the example herein was trained on 380000 input vectors which were each labelled with a spatiotemporal dispersion value, and tested on more than 110000 input vectors.

In some embodiments, the CRI 122 of CP 120 is configured such that when executed by PC 102, the CRI 122 causes the apparatus 2 to perform steps described herein (e.g., steps described herein with reference to the block diagrams). In other embodiments, the apparatus may be configured to perform steps described herein without the need for code. That is, for example, PC 102 may consist merely of one or more ASICs. Hence, the features of the embodiments described herein may be implemented in hardware and/or software.

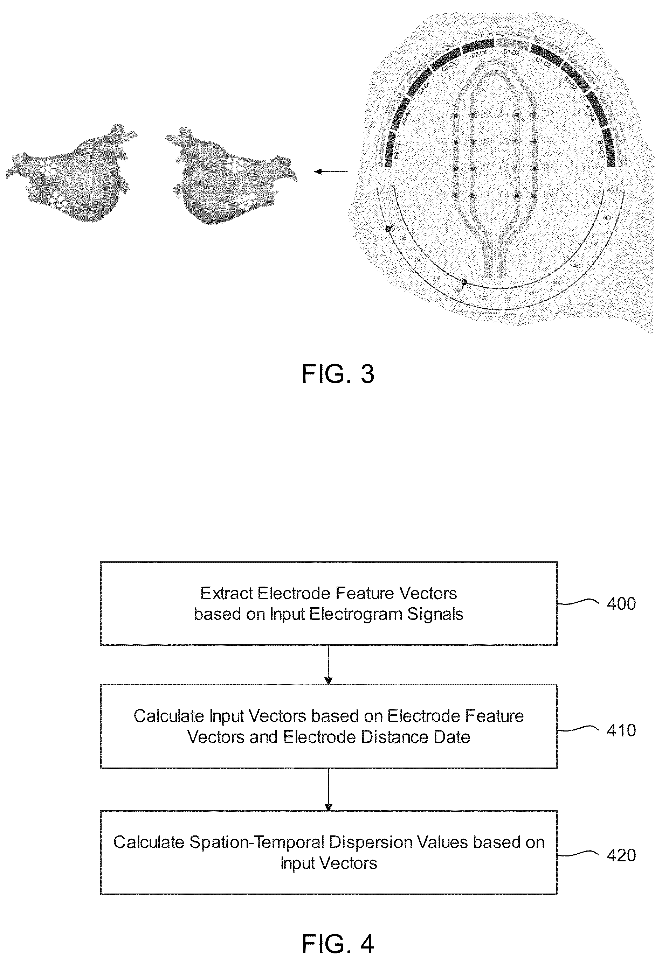

Based on the results of the spatiotemporal dispersion value, the apparatus 2 may further determine colors based on the prediction array. More precisely, this determination can be based by using prediction arrays associated with consecutive timecodes. If it is determined that the atrial fibrillation of the patient is slow, two prediction arrays corresponding to consecutive timecodes can be averaged, and the values of this array be associated as follows: blue if the value is less than 0.35, orange if the value is comprised between 0.35 and 0.65, and else red. If. If it is determined that the atrial fibrillation of the patient is fast, four prediction arrays corresponding to consecutive timecodes can be averaged, and the values of this array be associated as follows: blue if the value is less than 0.5, orange if the value is comprised between 0.5 and 0.8, and else red. The determination of whether the atrial fibrillation is fast or slow is a practitioner decision, which he inputs in the device. This allows to provide a visual return on a display as shown on Figure 3, which may be used by the practitioner to tag automatically, manually or semi-manually the heart region.

Figure 4 shows an exemplary schematic block diagram of the operation of apparatus 2 according to an embodiment. The apparatus 2 includes the modules represented on Figure 2, each of which is implemented in software. These modules provide the functionality of apparatus 2 described herein (e.g., steps described herein).

In a first operation 400, a set of electrogram signals obtained from electrodes of a catheter and associated with the same timecode are fed to the first module, that is the random convolutional kernel-based feature extractor 1210. These electrogram signals are accompanied by electrode distance data which match the same timecode for these electrodes. The result is a set of electrode feature vectors, with one electrode feature vector per originating electrogram signal in the input set. It will be understood that the output does not need to be a vector *per se.* It may be provided in any form as long as the electrode feature vectors are recoverable and separable from one another.

After operation 400, the set of electrode feature vectors are fed, along with the electrode distance data to the second module, that is the input generator 1220, in an operation 410. As described above, the input generator 1220 provides, for each electrogram signal of operation 400, an input vector which comprises the electrode feature vector of each electrogram signal, as well as the electrode feature vectors of the at least three electrodes closest to the electrode from which is derived each electrode feature vector of operation 400, along with the corresponding electrode distance data.

Finally, in an operation 420, each input vector is sent to the third module, that is the gradient boosted machine learning module 1230 in order to calculate the spatiotemporal dispersion value of each electrode signal.

The tests performed by the Applicant have shown the following results:
VX1 (original implementation): precision 0.61 / recall 0.74 / F1 0.67
Embodiment of Figure 1: precision 0.69 / recall 0.72 / F1 0.70

The invention thus clearly is an improvement over the implementation of French patent application FR1852850.

While various embodiments of the present disclosure are described herein, it should be understood that they have been presented by way of example only, and not limitation. Thus, the breadth and scope of the present disclosure should not be limited by any of the above-described exemplary embodiments. Moreover, any combination of the above-described elements in all possible variations thereof is encompassed by the disclosure unless otherwise indicated herein or otherwise clearly contradicted by context.

Additionally, while the processes described above and illustrated in the drawings are shown as a sequence of steps, this was done solely for the sake of illustration. Accordingly, it is contemplated that some steps may be added, some steps may be omitted, the order of the steps may be re-arranged, and some steps may be performed in parallel.

Some embodiments concern a computer-implemented method for identifying a cardiac region of interest, the method comprising:
a) receiving, using a plurality of electrodes, a set of electrogram signals;
b) receiving a set of electrode distance data corresponding to the plurality of electrodes;
c) applying a random convolutional kernel-based feature extractor to the received set of electrogram signals to generate an electrode feature vector for each electrogram signal;
d) for each electrode feature vector generated, associating the generated electrode feature vectors for at least three electrodes closest in proximity based on the received set of electrode distance data and corresponding electrode distance data; and
e) for each electrode feature vector generated, applying a gradient boosted machine learning (ML) module to generate a spatiotemporal dispersion value, wherein the gradient boosted machine learning (ML) module is trained on data comprising sets of input vectors labelled with a value indicating, for each electrode, if its electrogram signal exhibits spatiotemporal dispersion.

In this method, the generated spatiotemporal dispersion values may be used for identifying a cardiac region of interest.

In this method, the set of electrogram signals received are provided in real-time during a procedure.

Additionally, some embodiments concern a computing device comprising:
processing circuitry; and
a memory containing instructions executable by the processing circuitry for identifying a cardiac region of interest, the computing device operative to:
   a) receive, using a plurality of electrodes, a set of electrogram signals;
   b) receive a set of electrode distance data corresponding to the plurality of electrodes;
   c) apply a random convolutional kernel-based feature extractor to the received set of electrogram signals to generate an electrode feature vector for each electrogram signal;
   d) for each electrode feature vector generated, associate the generated electrode feature vectors for at least three electrodes closest in proximity based on the received set of electrode distance data and corresponding electrode distance data; and
   e) for each electrode feature vector generated, apply a gradient boosted machine learning (ML) module to generate a spatiotemporal dispersion value, wherein the gradient boosted machine learning (ML) module is trained on data comprising sets of input vectors labelled with a value indicating, for each electrode, if its electrogram signal exhibits spatiotemporal dispersion.

In this computing device, the generated spatiotemporal dispersion values are used for identifying a cardiac region of interest.

In this computing device, the set of electrogram signals received may be provided in real-time during a procedure.

## Claims

1. A computer device for analysis of electrograms, comprising
a data storage (114) arranged to receive electrogram signals each originating from one of a plurality of electrodes of a catheter and catheter electrode distance data, a random convolutional kernel-based feature extractor (1210) arranged to receive electrogram signals associated with the electrodes of a catheter for a given timecode and to return an electrode feature vector for each electrogram signal, an input generator (1220) arranged to receive the electrogram feature vectors output by said random convolutional kernel-based feature extractor (1210) and catheter electrode distance data relating to the catheter from which these electrogram signals originate, to associate each electrode with at least the three closest electrodes of the catheter based on said catheter electrode distance data, and to return an input vector in which the electrode feature vectors of each electrode is grouped with the electrode feature vectors of its associated electrodes and the corresponding catheter electrode distance data, and a gradient-boosted based machine learning module (1230) trained on data comprising sets of input vectors labelled with a value indicating for each electrode if its electrogram signal exhibits spatiotemporal dispersion, the computer device being arranged to receive electrogram signals associated and electrode distance date associated with a timecode, to feed them to said input generator (1220), to use the resulting input vectors as input for said gradient-boosted based machine learning module (1230), and to return a dispersion value for each electrode.

2. Computer device according to claim 1, in which the computer device operates in real-time on electrogram signals and the electrode distance data acquired in real-time.

3. Computer device according to claim 1 or 2, in which the input generator (1220) is arranged to associate each electrode with at least the four closest electrodes of the catheter based on said catheter electrode distance data.

4. Computer device according to one of the preceding claims, in which the gradient boosted machine learning module (1230) is an XGB.

5. Computer device according to one of the preceding claims, further arranged to determine a color associated to the spatiotemporal dispersion values for each electrogram signal, the computer device further comprising a display arranged to output, for each electrode, the color associated to the spatiotemporal dispersion value determined for the corresponding electrogram signal.

6. Computer implemented method for analyzing electrogram signals and corresponding electrode distance data comprising the following operations:
a) Applying a random convolutional kernel-based feature extractor (400) to said electrogram signals to return an electrode feature vector for each input electrogram signal,
b) For each electrode feature vector of operation a), based on the electrode distance data, associating (410) electrode feature vectors of the at least three electrodes closest to the electrode from which is derived said each electrode feature vector of operation a), along with the corresponding electrode distance data,
c) For each input vector, applying (420) a gradient boosted machine learning module trained on data comprising sets of input vectors labelled with a value indicating for each electrode if its electrogram signal exhibits spatiotemporal dispersion, and returning the corresponding values.

7. Computer implemented method according to claim 6, which is performed operated in real-time on electrogram signals and the electrode distance data acquired in real-time.

8. Computer implemented method according to claim 6 or 7, in which operation b) is arranged to associate each electrode with at least the four closest electrodes of the catheter based on said catheter electrode distance data.

9. Computer implemented method according to one of claims 6 to 8, in which the gradient boosted machine learning module (1230) is an XGB.

10. Computer implemented method according to one of claims 6 to 9, further comprising d) determining a color associated to the spatiotemporal dispersion values for each electrogram signal, and outputting, for each electrode, the color associated to the spatiotemporal dispersion value determined for the corresponding electrogram signal.

11. A computer program (120) comprising instructions (122) which, when executed by processing circuitry (102), causes the processing circuitry to carry out the methods of any one of claims 6 to 10.

12. A carrier containing a computer program (120) according to claim 11 wherein the carrier comprises one of an electronic signals, optical signal, radio signal or computer readable storage medium.

13. A computer program product (116) comprising a non-transitory computer readable medium (118) having stored thereon a computer program (120) according to claim 12.

## Patentansprüche

1. Computervorrichtung zur Analyse von Elektrogrammen, umfassend einen Datenspeicher (114), der dafür eingerichtet ist, Elektrogrammsignale, die jeweils von einer aus einer Vielzahl von Elektroden eines Katheters stammen, und Katheterelektroden-Abstandsdaten zu empfangen, einen Merkmalsextraktor auf Basis eines zufälligen Faltungskerns (1210), der dafür eingerichtet ist, Elektrogrammsignale, die den Elektroden eines Katheters für einen gegebenen Zeitcode zugeordnet sind, zu empfangen und für jedes Elektrogrammsignal einen Elektrodenmerkmalsvektor zurückzugeben, einen Eingabegenerator (1220), der dafür eingerichtet ist, die von dem besagten Merkmalsextraktor auf Basis eines zufälligen Faltungskerns (1210) ausgegebenen Elektrogramm-Merkmalsvektoren und Katheterelektroden-Abstandsdaten bezüglich des Katheters, von dem diese Elektrogrammsignale stammen, zu empfangen, um jede Elektrode auf der Grundlage der besagten Katheterelektroden-Abstandsdaten mit mindestens den drei nächstgelegenen Elektroden des Katheters zu assoziieren und um einen Eingabevektor zurückzugeben, in dem die Elektrodenmerkmalsvektoren jeder Elektrode mit den Elektrodenmerkmalsvektoren ihrer zugehörigen Elektroden und den entsprechenden Katheterelektroden-Abstandsdaten gruppiert sind, und ein maschinelles Lernmodul auf Basis von Gradientenverstärkung (1230), das auf Daten trainiert ist, die Sätze von Eingabevektoren umfassen, die mit einem Wert gekennzeichnet sind, der für jede Elektrode angibt, ob ihr Elektrogrammsignal eine spatiotemporale Dispersion aufweist, wobei die Computervorrichtung dafür eingerichtet ist, zugeordnete Elektrogrammsignale und einem Zeitcode zugeordnete Elektroden-Abstandsdaten zu empfangen, sie dem besagten Eingabegenerator (1220) zuzuführen, die resultierenden Eingabevektoren als Eingabe für das besagte maschinelle Lernmodul auf Basis von Gradientenverstärkung (1230) zu verwenden und für jede Elektrode einen Dispersionswert zurückzugeben.

2. Computervorrichtung nach Anspruch 1, wobei die Computervorrichtung in Echtzeit mit Elektrogrammsignalen und den in Echtzeit erfassten Elektroden-Abstandsdaten arbeitet.

3. Computervorrichtung nach Anspruch 1 oder 2, wobei der Eingabegenerator (1220) dafür eingerichtet ist, jede Elektrode auf der Grundlage der besagten Katheterelektroden-Abstandsdaten mit mindestens den vier nächstgelegenen Elektroden des Katheters zu assoziieren.

4. Computervorrichtung nach einem der vorhergehenden Ansprüche, wobei das maschinelle Lernmodul auf Basis von Gradientenverstärkung (1230) ein XGB ist.

5. Computervorrichtung nach einem der vorhergehenden Ansprüche, ferner dafür eingerichtet, eine den spatiotemporalen Dispersionswerten für jedes Elektrogrammsignal zugeordnete Farbe zu bestimmen, wobei die Computervorrichtung ferner eine Anzeige umfasst, die dafür eingerichtet ist, für jede Elektrode die dem für das entsprechende Elektrogrammsignal bestimmten spatiotemporalen Dispersionswert zugeordnete Farbe auszugeben.

6. Computerimplementiertes Verfahren zur Analyse von Elektrogrammsignalen und entsprechenden Elektroden-Abstandsdaten, umfassend die folgenden Operationen:
a) Anwenden eines Merkmalsextraktors auf Basis eines zufälligen Faltungskerns (400) auf die besagten Elektrogrammsignale, um einen Elektrodenmerkmalsvektor für jedes Eingangselektrogrammsignal zurückzugeben;
b) für jeden Elektrodenmerkmalsvektor der ersten Operation, basierend auf den Elektroden-Abstandsdaten, Assoziieren (410) von Elektrodenmerkmalsvektoren von mindestens den drei Elektroden, die der Elektrode am nächsten liegen, von der jeder besagte Elektrodenmerkmalsvektor der ersten Operation abgeleitet ist, zusammen mit den entsprechenden Elektroden-Abstandsdaten;
c) für jeden Eingabevektor, Anwenden (420) eines maschinellen Lernmoduls auf Basis von Gradientenverstärkung, das auf Daten trainiert ist, die Sätze von Eingabevektoren umfassen, die mit einem Wert gekennzeichnet sind, der für jede Elektrode angibt, ob ihr Elektrogrammsignal eine spatiotemporale Dispersion aufweist, und Zurückgeben der entsprechenden Werte.

7. Computerimplementiertes Verfahren nach Anspruch 6, das in Echtzeit mit Elektrogrammsignalen und den in Echtzeit erfassten Elektroden-Abstandsdaten betrieben wird.

8. Computerimplementiertes Verfahren nach Anspruch 6 oder 7, wobei die Operation b) dafür eingerichtet ist, jede Elektrode auf der Grundlage der besagten Katheterelektroden-Abstandsdaten mit mindestens den vier nächstgelegenen Elektroden des Katheters zu assoziieren.

9. Computerimplementiertes Verfahren nach einem der Ansprüche 6 bis 8, wobei das maschinelle Lernmodul auf Basis von Gradientenverstärkung (1230) ein XGB ist.

10. Computerimplementiertes Verfahren nach einem der Ansprüche 6 bis 9, ferner umfassend d) das Bestimmen einer den spatiotemporalen Dispersionswerten für jedes Elektrogrammsignal zugeordneten Farbe und das Ausgeben, für jede Elektrode, der dem für das entsprechende Elektrogrammsignal bestimmten spatiotemporalen Dispersionswert zugeordneten Farbe.

11. Computerprogramm (120), das Anweisungen (122) umfasst, die bei Ausführung durch eine Verarbeitungsschaltung (102) die Verarbeitungsschaltung veranlassen, die Verfahren nach einem der Ansprüche 6 bis 10 auszuführen.

12. Träger, der ein Computerprogramm (120) nach Anspruch 11 enthält, wobei der Träger eines von elektronischen Signalen, einem optischen Signal, einem Funksignal oder einem computerlesbaren Speichermedium umfasst.

13. Computerprogrammprodukt (116), das ein nicht-transitorisches computerlesbares Medium (118) umfasst, auf dem ein Computerprogramm (120) nach Anspruch 12 gespeichert ist.

## Revendications

1. Dispositif informatique pour l'analyse d'électrogrammes, comprenant une stockage de données (114) agencée pour recevoir des signaux d'électrogramme provenant chacun d'une d'une pluralité d'électrodes d'un cathéter et des données de distance d'électrode de cathéter, un extracteur de caractéristiques à base de noyau convolutif aléatoire (1210) agencé pour recevoir des signaux d'électrogramme associés aux électrodes d'un cathéter pour un marqueur de tempsdonné et pour renvoyer un vecteur de caractéristiques d'électrode pour chaque signal d'électrogramme, un générateur d'entrée (1220) agencé pour recevoir les vecteurs de caractéristiques d'électrogramme délivrés en sortie par ledit extracteur de caractéristiques à base de noyau convolutif aléatoire (1210) et des données de distance d'électrode de cathéter relatives au cathéter d'où proviennent ces signaux d'électrogramme, pour associer chaque électrode à au moins les trois électrodes les plus proches du cathéter sur la base desdites données de distance d'électrode de cathéter, et pour renvoyer un vecteur d'entrée dans lequel les vecteurs de caractéristiques d'électrode de chaque électrode sont groupés avec les vecteurs de caractéristiques d'électrode de ses électrodes associées et les données de distance d'électrode de cathéter correspondantes, et un module d'apprentissage automatique à base de gradient boosting (1230) entraîné sur des données comprenant des ensembles de vecteurs d'entrée étiquetés avec une valeur indiquant pour chaque électrode si son signal d'électrogramme présente une dispersion spatiotemporelle, le dispositif informatique étant agencé pour recevoir des signaux d'électrogramme associés et des données de distance d'électrode associées à un marqueur de temps, pour les fournir audit générateur d'entrée (1220), pour utiliser les vecteurs d'entrée résultants en tant qu'entrée pour ledit module d'apprentissage automatique à base de gradient boosting (1230), et pour renvoyer une valeur de dispersion pour chaque électrode.

2. Dispositif informatique selon la revendication 1, dans lequel le dispositif informatique fonctionne en temps réel sur des signaux d'électrogramme et les données de distance d'électrode acquises en temps réel.

3. Dispositif informatique selon la revendication 1 ou 2, dans lequel le générateur d'entrée (1220) est agencé pour associer chaque électrode à au moins les quatre électrodes les plus proches du cathéter sur la base desdites données de distance d'électrode de cathéter.

4. Dispositif informatique selon l'une des revendications précédentes, dans lequel le module d'apprentissage automatique à base de gradient boosting (1230) est un XGB.

5. Dispositif informatique selon l'une des revendications précédentes, agencé en outre pour déterminer une couleur associée aux valeurs de dispersion spatiotemporelle pour chaque signal d'électrogramme, le dispositif informatique comprenant en outre un affichage agencé pour délivrer en sortie, pour chaque électrode, la couleur associée à la valeur de dispersion spatiotemporelle déterminée pour le signal d'électrogramme correspondant.

6. Procédé mis en œuvre par ordinateur pour l'analyse de signaux d'électrogramme et de données de distance d'électrode correspondantes, comprenant les opérations suivantes consistant à :
a) appliquer un extracteur de caractéristiques à base de noyau convolutif aléatoire (400) auxdits signaux d'électrogramme pour renvoyer un vecteur de caractéristiques d'électrode pour chaque signal d'électrogramme d'entrée ;
b) pour chaque vecteur de caractéristiques d'électrode de la première opération, sur la base des données de distance d'électrode, associer (410) des vecteurs de caractéristiques d'électrode des au moins trois électrodes les plus proches de l'électrode à partir de laquelle est dérivé ledit chaque vecteur de caractéristiques d'électrode de la première opération, conjointement avec les données de distance d'électrode correspondantes ;
c) pour chaque vecteur d'entrée, appliquer (420) un module d'apprentissage automatique à base de gradient boosting entraîné sur des données comprenant des ensembles de vecteurs d'entrée étiquetés avec une valeur indiquant pour chaque électrode si son signal d'électrogramme présente une dispersion spatiotemporelle, et renvoyer les valeurs correspondantes.

7. Procédé mis en œuvre par ordinateur selon la revendication 6, qui est mis en œuvre en temps réel sur des signaux d'électrogramme et les données de distance d'électrode acquises en temps réel.

8. Procédé mis en œuvre par ordinateur selon la revendication 6 ou 7, dans lequel l'opération b) est agencée pour associer chaque électrode à au moins les quatre électrodes les plus proches du cathéter sur la base desdites données de distance d'électrode de cathéter.

9. Procédé mis en œuvre par ordinateur selon l'une des revendications 6 à 8, dans lequel le module d'apprentissage automatique à base de gradient boosting (1230) est un XGB.

10. Procédé mis en œuvre par ordinateur selon l'une des revendications 6 à 9, comprenant en outre d) la détermination d'une couleur associée aux valeurs de dispersion spatiotemporelle pour chaque signal d'électrogramme, et la sortie, pour chaque électrode, de la couleur associée à la valeur de dispersion spatiotemporelle déterminée pour le signal d'électrogramme correspondant.

11. Programme informatique (120) comprenant des instructions (122) qui, lorsqu'elles sont exécutées par un circuit de traitement (102), amènent le circuit de traitement à exécuter les procédés de l'une quelconque des revendications 6 à 10.

12. Support contenant un programme informatique (120) selon la revendication 11, dans lequel le support comprend l'un parmi des signaux électroniques, un signal optique, un signal radio ou un support de stockage lisible par ordinateur.

13. Produit-programme informatique (116) comprenant un support non transitoire lisible par ordinateur (118) sur lequel est stocké un programme informatique (120) selon la revendication 12.
